# EUROPEAN PATENT SPECIFICATION

(11) **EP 0 421 733 B1**
(45) Date of publication and mention of the grant of the patent: **05.04.1995**
(21) Application number: 90310779.5
(22) Date of filing: 02.10.1990
(51) Int. Cl.: A61K 31/71, A61K 9/10, A61K 9/127

(54) **Amphotericin B lipid complex**
Amphotericin-B-Lipidkomplex
Complexe lipidique d'amphotéricine B

(30) Priority: 02.10.1989 US 415801
(43) Date of publication of application: 10.04.1991
(73) Proprietor: THE LIPOSOME COMPANY, INC., Princeton, NJ 08540 (US)
(72) Inventor: Bonner, Daniel P., Hamilton Square, New Jersey (US); Clark, Junius M., Yardley, PA (US)
(74) Representative: Thomas, Roger Tamlyn

(56) References cited:
- 89TH ANNUAL MEETING OF THE AMERICAN SOCIETY FOR MICROBIOLOGY 1989, New Orleans,LA, 14th - 18th May 1989, page 7, abstract no. A-41; J.M. CLARK et al.:"Amphotericin B lipid complex: Efficacy in localized Candida infections inmice"
- CANCER DRUG DELIVERY, vol. 2, no. 3, summer 1985, pages 183-189, Mary AnnLiebert, Inc., Publishers, New York, US; G. LOPEZ-BERESTEIN et al.: "Protectiveeffect of liposomal-amphotericin B against C. Albicans infection in mice"

## Description

Amphotericin B is a broad spectrum polyene antifungal agent that has been shown to provide effective treatment for most mycotic infections. A complete description of this antibiotic can be found in U.S. 2,908,611. However, the utility of amphotericin B has been limited by its toxicity. Long term therapy is often associated with renal toxicity and anemia, and its acute toxicity include fever, chills, nausea and vomiting. In order to overcome this toxicity, a number of approaches have been developed. These generally include initiation of therapy with graded doses of amphotericin B to determine tolerance and close monitoring of renal function. Amphotericin B is currently available as a collodial suspension for administration by infusion. Use of sodium desoxycholate is necessary to achieve a colloidal suspension of amphotericin B which is otherwise insoluble or poorly soluble in agueous solution. It is believed, when given *in vivo* amphotericin B is rapidly bound to lipoproteins and red blood cell membranes.

Recently, Lopez-Berestein et al. have reported a new approach to the formulation of amphotericin B which results in lower toxicity while retaining or improving on its efficacy. This approach involves the encapsulation of amphotericin B in liposomes. These results have been reported in the following articles:
1. Lopez-Berestein, G., Mehta, R., Hopfa, R.L., et al. Treatment and Prophylaxis of Disseminated Infection Due to *Candida albicans* in Mice with Liposome-Encapsulated Amphotericin B. Brit. J. Infect. Dis. 147: 939-944; 1983.
2. Lopez-Berestein, G., Fainstein, V., Hopfer, R.L., et al. Liposomal Amphotericin B for the Treatment of Systemic Fungal Infections in Patients with Cancer: A Preliminary Report. J. Inf. Dis. 4: 704-710; 1985.
3. Lopez-Berestein, G., Bodey, G.P., Frankel, L.S., Mehta, R. Treatment of Hepatosplenic Fungal Infections with Liposomal Amphotericin B. Br. J. Clin. Oncol. 5: 310-317: 1987.
4. Lopez-Berestein et al. Protective effect of liposomal amphotericin B against Candida albicans infections in mice: Cancer Drug Delivery 2, 183-189(1985).

Treatment of Candida infections by an amphotericin B lipid complex in mice is also disclosed in 89th Annual Meeting of the American Society for Microbiology 1989, New Orleans, LA, 14th-18th May 1989, page 7, abstract A-41.

Most recently U.S. patent 4,663,167 has been issued and describes compositions of amphotericin B encapsulated within a liposome consisting essentially of dimyristoyl phosphatidylcholine and dimyristoyl phosphatidylglycerol in an approximately 7 to 3 molar ratio.

Also, European Patent Application EP-A-282405 describes compositions of lipids and amphotericin B which have a higher ratio of amphotericin B to lipids.

Up until now, systemic fungal infections in man have required prolonged, multiple treatments with the appropriate antifungal agents. Also, with increased use of immunosuppressive agents in organ transplantation and more aggressive chemotherapy in cancer patients, infections caused by opportunistic pathogens like *Candida* are becoming more prevalent.

Recently, fluconazole has received approval in England for one day therapy for the treatment of *Candida* vaginitis.

It has now been discovered that an amphotericin B lipid complex (ABLC) can be used to treat systemic *Candida* infections with a single dose administration. These include in-line catheter related candidemia, non-invasive *Candida* esophagitis and mucosal candidiasis. The amphotericin B lipid complex has relatively high ratios of amphotericin B to lipids as described in European Patent Application EP-A-282405.

A single dose administration makes the taking of ABLC extremely simple by eliminating the need for multiple administration.

A single dose administration of ALBC to treat *Candida* infections will provide an effectiveness that is at least equivalent or even greater than that achieved with the use of multiple doses of amphotericin B when the toxicity of amphotericin B is considered.

Single dose administration reduces the cost of therapy which can be high due to the long duration of treatment with multiple doses.

Single dose administration reduces the probability of side-effects occurring by reducing the amount of amphotericin B necessary to treat *Candida* infection.

Fungal infections in man in general and *Candida* infections specifically require prolonged, multiple treatments with the appropriate antifungal agent. This invention provides a method of treating *Candida* infections in mammals which require only one dose. This is possible because of the unique properties of the amphotericin lipid complex.

The amphotericin B lipid complex of this invention is prepared by dissolving amphotericin B in DMSO, filtering and sterilizing by further filtration. This filtered and sterilized solution is aseptically combined with a filter-sterilized solution of Dimyristoyl phosphatidylcholine and, Dimyristoyl phosphatidylglycerol in methylene chloride in a sterile mixing vessel. Filter-sterilized sodium chloride injection (normal saline) is added to the above mixture in order to hydrate the complex. The methylene chloride portion of the mixture is removed by evaporation. The remaining sterile suspension of ABLC complex in saline, with residual DMSO, is further diluted with sterile normal saline for ease of handling. The suspension is aseptically milled via a sterilized colloid mill until the bulk of the product is below five microns in particle size. The milled suspension is aseptically filtered through ceramic tangential filters of five micron and 1.2 micron pore size in order to recover the fraction of drug-lipid complex between one and five microns.

The ABLC formulations described in this invention can be used to treat various fungal-related diseases in mammals such as in-line catheter related candidemia, non-invasive *Candida* esophagitis and mucosal candidiasis by a single-dose administration. For combating fungal infections in mammals a formulation of this invention can be administered as a single dose in an amount of about 0.5 mg/kg to about 10 mg/kg. The preferred mode of administration is the intravenous route. The following examples illustrate that a single dose administration is effective in treating systemic candidiasis in mice.

### Example 1

The following ABLC composition was prepared in the manner described above.

| | |
|---|---|
| Amphotericin B | 5.0 mg |
| Dimyristoyl phosphatidylcholine (DMPC) | 4.6 mg |
| Dimyristoyl phosphatidylglycerol (DMPG) | 2.0 mg |
| Sodium Chloride | 9.0 mg |
| Water for Injection, enough to make | 1.0 ml |

### Example 2

Female Swiss Webster mice were infected intraveneously with 5 x 10⁶ blastospores of *C*. albicans SC 5314 and treated one time with either ABLC or amphotericin B, five hours later. The amphotericin B was diluted in 5% dextrose for intravenous injection into the experimental mice. The ABLC formulation was also administered intravenously. The results are reproduced in Table 1 appearing below:

| Preparation | ED₅₀(mg AMB/kg) | |
|---|---|---|
| | Survival | Clearance |
| ABLC | 1.16 (1.07 - 1.28) | 1.93 (1.19 - 2.8) |
| Amphotericin B | 0.46 (0.36 - 0.54) | 0.7 (0.54 - >0.8) |

Table 1 represents a range of three experiments. As noted in Table 1, ABLC is efficacious with a survival ED₅₀ of 1.16 mg/kg of ABLC and a range of from 1.07 to 1.28 for the three experiments represented compared to 0.46 for amphotericin B, range from 0.36 to 0.54. When efficacy is based on kidney clearance, ABLC is still efficacious with an ED₅₀ of 1.93 compared to 0.7 with amphotericin B.

## Claims

1. Use of Amphotericin B Lipid Complex for the manufacture of a medicament for the treatment of systemic fungal infections in mammals in a single administration.

2. Use according to claim 1 wherein the amount to be administered is 0.5 mg/kg to about 10 mg/kg.

3. Use according to claim 1 wherein the fungal infection is inline catheter related candidemia.

4. Use according to claim 1 wherein the fungal infection is Candida esophogitis.

5. Use according to claim 1 wherein the fungal infection is mucosal candidiasis.

## Patentansprüche

1. Verwendung von Amphotericin B-Lipidkomplex zur Herstellung eines Medikamentes zur Behandlung von systemischen fungalen Infektionen bei Säugern in einer einmaligen Verabreichung.

2. Verwendung nach Anspruch 1, worin die zu verabreichende Menge 0,5 mg/kg bis etwa 10 mg/kg beträgt.

3. Verwendung nach Anspruch 1, worin die fungale Infektion Dauerkatheter-Candidämie (in-line katheter related candedemia) ist.

4. Verwendung nach Anspruch 1, worin die fungale Infektion Candida Ösophagitis ist.

5. Verwendung nach Anspruch 1, worin die fungale Infektion mucosale Candidiasis ist.

## Revendications

1. Utilisation de complexe lipidique d'amphotéricine B pour la préparation d'un médicament pour le traitement d'infections fongiques générales chez les mammifères en une administration unique.

2. Utilisation selon la revendication 1, dans laquelle la quantité à administrer est de 0,5 mg/kg à environ 10 mg/kg.

3. Utilisation selon la revendication 1, dans laquelle l'infection fongique est une candidémie se rapportant à un cathéter en place.

4. Utilisation selon la revendication 1, dans laquelle l'infection fongique est une Candida esophagitis.

5. Utilisation selon la revendication 1, dans laquelle l'infection fongique est une candidose des muqueuses.
